# EUROPÄISCHE PATENTANMELDUNG

(11) **EP 2 260 883 A2**
(43) Veröffentlichungstag der Anmeldung: **15.12.2010**
(21) Anmeldenummer: 10160054.2
(22) Anmeldetag: 15.04.2010
(51) Int. Cl.: A61L 31/02, A61L 31/14, A61N 1/00

(54) **Verfahren und Vorrichtung zur Beeinflussung und Steuerung der Korrosionsrate von Implantaten**

(30) Priorität: 09.06.2009 EP 09162307
(71) Anmelder: Heller, Jorg, 51766 Engelskirchen (DE)
(72) Erfinder: Heller, Jörg, 51766, Engelskirchen (DE); Dr. Sieber, Irina, 85051, Ingolstadt (DE); Oberhauser, Simon, 85296, Rohrbach (DE)
(74) Vertreter: Bergmeier, Werner

(57) **Zusammenfassung**

Bei einem Verfahren zur Beeinflussung und zur Steuerung der Korrosionsrate von Implantaten (2) aus einem metallischen, biokompatiblen und biokorrodierbaren Werkstoff in vivo, wird eine elektrochemische Zelle mit oder ohne direkter Verbindung zu Körpergewebe (5) des Patienten als Elektrolyt aufgebaut. Es wird eine Spannungs- oder Stromquelle (4) außerhalb des Körpers des Patienten vorgesehen und wenigstens eine Elektrode (3) und/oder eine elektrisch leitende Verbindung (15) nach Implantation des Implantats (2) an dem oder in das Körpergewebe (5) appliziert. Die wenigstens eine Elektrode (3) und/oder die elektrisch leitende Verbindung (15) wird entweder direkt kontaktierend oder mittels Streuströmen über Körpergewebe (5) des Patienten mit dem Implantat (2) verbunden. Nach Implantation eines Implantats (2) erfolgt eine gezielte Steuerung einer Auflösung oder Stabilisierung des Implantats (2) durch Veränderung der Potentiallage.

Eine entsprechende Vorrichtung (1) umfasst ein Implantat (2) aus einem metallischen, biokompatiblen und biokorrodierbaren Werkstoff, und zwei Elektroden (3), welche jeweils elektrisch leitend permanent oder entsprechend dem Genesungszustand des Patienten mit einer Strom- oder Spannungsquelle (4) verbunden sind. Die Strom- oder Spannungsquelle (4) befindet sich außerhalb des Körpers des Patienten und wenigstens eine Elektrode (3) und/oder eine elektrisch leitende Verbindung (15) ist nach Implantation des Implantats (2) von außen an dem oder in das Körpergewebe (5) applizierbar. Die wenigstens eine Elektrode (3) und/oder die elektrisch leitende Verbindung (15) ist entweder direkt kontaktierend oder mittels Streuströmen über Körpergewebe (5) des Patienten mit dem Implantat (2) verbunden.

## Beschreibung

Die vorliegende Erfindung betrifft ein Verfahren zur Beeinflussung der Korrosionsrate von Implantaten aus einem metallischen biokompatiblen und biokorrodierbaren Werkstoff in vivo. Hierbei werden zwei Elektroden elektrisch leitend mit einer Strom- und Spannungsquelle verbunden und es wird eine elektrochemische Zelle mit Körpergewebe des Patienten als Elektrolyt aufgebaut. Die Korrosionsrate des Implantates wird durch Anlegen eines Stromes oder einer Spannung beeinflusst. Weiterhin betrifft die Erfindung eine entsprechende Vorrichtung zur Beeinflussung der Korrosionsrate von Implantaten.

Medizinische Implantate sind in verschiedenen Ausgestaltungen aus dem Stand der Technik bekannt. Zu unterscheiden sind hierbei permanente Implantate, welche zum dauerhaften Verbleib im menschlichen Körper bestimmt sind und temporäre Implantate, welche nur zum zeitweisen Verbleib im Körper ausgebildet sind. Diese Implantate müssen, nachdem sie ihren Zweck erfüllt haben, in der Regel durch einen zweiten operativen Eingriff wieder aus dem Körper entfernt werden, da es zu unerwünschten Reaktionen des Körpers auf dem Fremdkörper kommen könnte und somit lokale Infektionsgefahr besteht. Als temporäre medizinische Implantate sind beispielsweise chirurgische bzw. orthopädische Schrauben oder Platten, Gefäßprothesen, Stents sowie Wirkstoffdepots und weitere bekannt.

Um die aufwendige operative Entfernung von Implantaten zu vermeiden, ist es bereits bekannt geworden, Implantate aus biokorrodierbaren bzw. biodegradierbaren und im Körper abbaubaren Werkstoffen herzustellen. Bekannt geworden sind biodegradierbare Implantate aus Kunststoffmaterialien wie beispielsweise in der DE 2502884 C2 beschrieben, welche in erster Linie für Stents verwendet werden.

Metallische Werkstoffe sind zwar prinzipiell biokorrodierbar, es bestehen jedoch erhebliche Unterschiede in den Korrosionsgeschwindigkeiten. Als temporäre Implantate sind beispielsweise Stents aus Magnesium-EisenLegierungen bekannt. Diese korrodieren in vivo mit derart hohen Korrosionsraten, dass eine Explantation nicht erforderlich ist. Häufig werden auch Magnesiumlegierungen als Implantate verwendet, da diese in vivo schnell korrodieren und nur geringe Toxizität aufweisen. Die Korrosionsraten sind jedoch vergleichsweise hoch, so dass die Implantate oftmals zu schnell korrodieren. Weiterhin sind biokompatible Legierungen wie beispielsweise Magnesium-Aluminium oder Magnesium-Calcium bekannt. Diese unterliegen einem gleichmäßigen Angriff und sind damit besser geeignet, aber auch hier ist eine weitere Erhöhung des Korrosionswiderstands wünschenswert. Die Möglichkeit, die Korrosionsgeschwindigkeit des Implantates in vivo durch Zusatz bestimmter Legierungselemente sowie Abstimmung der Materialstärke des Werkstoffes zu beeinflussen, wird beispielsweise in der EP 1 270 023 B1 beschrieben.

Weiterhin ist es bekannt geworden, auf Magnesiumimplantate eine Korrosionsschutzschicht aufzubringen, um die Korrosionsrate des Implantates in vivo zu beeinflussen. Die DE 10 2006 038 231 A1 schlägt beispielsweise vor, das Implantat mit einer Beschichtung aus einer Organo-Silizium-Verbindung zu überziehen, welche die Korrosionsrate verzögert, so dass die Implantate auch länger beständig bleiben. Die DE 10 2006 060 501 A1 schlägt hingegen vor, ein Magnesiumimplantat mit einer wässrigen oder alkoholischen Konversionslösung zu behandeln, um eine korrosionshemmende Schicht zu erzielen.

Eine Beeinflussung des Korrosionsverhaltens von Implantaten ist somit über die Wahl der Werkstoffe und ihrer Legierungsbestandteile oder durch das Aufbringen bestimmter Beschichtungen in eingeschränktem Maß möglich. Bei schnell korrodierenden Materialen wie Magnesium wird häufig trotz aufgebrachter Beschichtungen das Implantat vergleichsweise schnell aufgelöst und unzulässig geschwächt, so dass ein Implantat auf Magnesiumbasis nur in bestimmten Fällen einsetzbar ist. Materialien wie Stahl weisen gegenüber Magnesiumwerkstoffen oftmals günstigere mechanische Eigenschaften auf, korrodieren jedoch in vivo zu langsam, so dass eine erneute Operation zur Entfernung des Implantates erforderlich ist.

Um eine gezielte Beeinflussung der Korrosionsrate eines Implantates in vivo zu erreichen, schlägt die WO 2008/034050 A2 vor, einen Stent aus einem biokorrodierbaren Material mit einer Vielzahl magnetischer Induktionspartikel zu versehen. Der implantierte Stent wird einem Magnetfeld ausgesetzt, so dass die magnetischen Induktionspartikel in Bewegung versetzt werden und hierdurch die Korrosionsrate beeinflusst werden kann. Die Herstellung eines derartigen Implantates ist durch die Einlagerung der Induktionspartikel vergleichsweise aufwändig. Zudem ist diese auf bestimmte Materialien beschränkt und die magnetischen Partikel müssen ggf. mit einer Beschichtung versehen werden.

Die DE 100 17 642 A1 befasst sich mit dem Schutz permanenter Implantate, wie z. B. Schrittmacher, Nervenstimulatoren oder Muskelstimulatoren, bei welchen eine Explantation nicht vorgesehen ist. Um die Oberfläche der Implantate vor dem sie umgebenden korrosiven Fluid zu schützen, kann eine Pulswellenform an die Oberfläche des implantierten Gerätes zugeführt werden. Das implantierbare Gerät beinhaltet hierzu einen Pulswellengenerator sowie zwei Elektroden. Eine der Elektroden ist direkt mit der Oberfläche des Implantates verbunden und ein geschlossener Stromkreis wird durch das korrosive Fluid erzeugt. Eine Beeinflussung der Vorgänge nach Implantation ist nicht möglich.

Aufgabe der vorliegenden Erfindung ist es daher, eine Vorrichtung und ein Verfahren vorzuschlagen, welche eine gezielte Beeinflussung der Korrosionsrate von Implantaten in vivo von außen ermöglichen und auch für temporäre Implantate geeignet sind.

Die Aufgabe wird gelöst mit den Merkmalen der unabhängigen Ansprüche.

Ein Verfahren zur Beeinflussung der Korrosionsrate von Implantaten aus einem metallischen biokompatiblen und biokorrodierbaren Werkstoff in vivo sieht vor, dass eine elektrochemische Zelle mit Körpergewebe des Patienten als Elektrolyt aufgebaut wird. Im Rahmen der vorliegenden Erfindung sollen unter dem Begriff "Körpergewebe" sämtliche verschiedenen Gewebe des Körpers sowie sämtliche Körperflüssigkeiten unabhängig vom Grad der Durchblutung bzw. unabhängig von der Fließgeschwindigkeit verstanden werden. Erfindungsgemäß erfolgt bei dem Verfahren bzw. mit der entsprechenden Vorrichtung eine gezielte Steuerung einer Auflösung oder einer Stabilisierung des Implantates nach Implantation außerhalb des Körpers des Patienten. Hierzu wird eine Strom- oder Spannungsquelle außerhalb des Körpers des Patienten vorgesehen und wenigstens eine Elektrode und/oder eine elektrisch leitende Verbindung wird bereits während oder nach Implantation des Implantats an dem oder in das Körpergewebe appliziert. Eine entsprechende Vorrichtung umfasst zwei Elektroden sowie eine Strom- oder Spannungsquelle, um mit Körpergewebe des Patienten als Elektrolyt eine elektrochemische Zelle aufzubauen. Die Elektroden sind hierbei elektrisch leitend mit der Strom- oder Spannungsquelle verbunden. Erfindungsgemäß ist die Strom- oder Spannungsquelle außerhalb des Körpers des Patienten angeordnet und wenigstens eine Elektrode und/oder eine elektrisch leitende Verbindung ist während oder nach der Implantation des Implantates von außen an das Körpergewebe oder in das Körpergewebe applizierbar. Die wenigstens eine Elektrode und/oder die elektrisch leitende Verbindung ist bzw. wird entweder direkt kontaktierend oder ausschließlich über Körpergewebe des Patienten mittels Streuströmen mit dem Implantat verbunden.

Durch die vorgeschlagene Anordnung ist es möglich, die Korrosionsrate des Implantates in vivo nach Implantation gezielt durch Anliegen eines Stromes oder einer Spannung zu beeinflussen. Mit dem erfindungsgemäßen Verfahren bzw. der erfindungsgemäßen Vorrichtung ist es durch die Ausbildung als elektrochemische Zelle möglich, Implantate bestehend aus einem Werkstoff mit einer hohen Korrosionsrate wie z. B. Magnesium gezielt zu stabilisieren, um die Verweildauer im Körper zu erhöhen. Ebenso kann mit dem erfindungsgemäßen Verfahren die Korrosionsgeschwindigkeit vergleichsweise langsam korrodierender Implantatwerkstoffe, beispielsweise Eisenwerkstoffen, gezielt erhöht werden. Die gezielte Steuerung der Korrosionsrate ist hierbei durch eine Verschiebung der Potentiallage von degradierbaren Werkstoffen möglich. Das Implantat kann hierbei beliebige, einsatzangepasste Eigenschaften aufweisen.

Vorteilhafterweise wird bei dem erfindungsgemäßen Verfahren ein Stromkreis der elektrochemischen Zelle erst nach Implantation geschlossen. Ebenso kann jedoch auch bei sehr schnell korrodierenden Implantatwerkstoffen der Stromkreis bereits während der Implantation geschlossen werden. Neben dem Implantat selbst müssen daher keine weiteren Teile, welche den Patienten belasten könnten, oder später in einer aufwendigen zweiten Operation explantiert werden müssten, implantiert werden. Zudem ist die Erfindung universell sowohl zur Stabilisierung wie auch zur Auflösung von verschiedensten Implantaten aus verschiedensten Werkstoffen einsetzbar, da eine gezielte Steuerung von außerhalb des Patienten erfolgt. Die Korrosionsrate kann hierbei je nach Höhe der angelegten Spannung bzw. der Stromstärke erheblich verringert oder erhöht werden. Zudem ist es möglich, die Korrosionsrate konstant einzustellen oder jeweils den Erfordernissen entsprechend anzupassen.

Nach einer ersten Ausführung der Erfindung werden nach Implantation des Implantates außerhalb des Körpers des Patienten zwei Elektroden vorgesehen. Die Elektroden werden vorzugsweise auf der Haut des Patienten derart platziert, dass das Implantat in vivo zwischen den beiden Elektroden liegt, und die beiden Elektroden werden ausschließlich über Körpergewebe des Patienten mit dem Implantat verbunden. Die Stabilisierung oder Auflösung des Implantats erfolgt ohne direkte Kontaktierung des Implantats ausschließlich durch das Implantat durchfließende Streuströme. Durch die erfindungsgemäße Ausbildung der Vorrichtung, bei welcher nach Implantation des Implantats keinerlei invasive Eingriffe mehr erforderlich sind, kann eine Auflösung oder Stabilisierung des Implantates besonders schonend vorgenommen werden. Überraschenderweise hat sich herausgestellt, dass die Auflösung oder Stabilisierung des Implantats mittels Streuströmen kontrollierbar und steuerbar ist. Vorzugsweise werden die Elektroden hierbei in Bezug auf das Implantat derart platziert, dass durch den Stromfluss zwischen den Elektroden in dem Implantat ein anodischer und ein kathodischer Bereich ausgebildet wird. Dies ermöglicht eine gezielte Einstellung der Korrosionsrate des Implantats. Die Korrosionsgeschwindigkeit stellt sich hierbei abhängig davon ein, ob in dem Implantat der kathodische oder der anodische Bereich überwiegt.

Vorzugsweise handelt es sich bei dem Implantat um ein temporäres Implantat aus einem biokompatiblen und biokorrodierbaren Werkstoff, da bei diesem entsprechend der jeweils vorherrschenden Bedürfnisse eine gezielte Einstellung der Korrosionsrate und somit der Lebensdauer des Implantates gewünscht ist.

Nach einer anderen Weiterbildung der Erfindung wird nach Implantation des Implantates die Spannungs- oder Stromquelle über eine elektrisch leitende Verbindung direkt mit dem Implantat als Arbeitselektrode und der zweiten Elektrode als Gegenelektrode verbunden. Die Arbeitselektrode und die Gegenelektrode werden über Körpergewebe des Patienten als Elektrolyt verbunden. Die Gegenelektrode und die elektrisch leitende Verbindung werden hierbei perkutan in das Körpergewebe eingebracht, so dass der Stromkreis der elektrochemischen Zelle erst nach Implantation des Implantats geschlossen wird. Auch hier können zu einem beliebigen gewünschten Zeitpunkt nach Implantation die Elektroden appliziert werden und der Stromkreis geschlossen werden und somit eine gezielte Steuerung der Korrosionsrate des Implantates in vivo von außerhalb des Körpers des Patienten vorgenommen werden. Die Elektroden bzw. die leitende Verbindung sind beispielsweise als Einstichnadeln ausgebildet, welche problemlos auch bis in tiefere Gewebeschichten eingebracht werden können.

Nach einer anderen Weiterbildung der Erfindung ist das Implantat und/oder die Gegenelektrode implantierbar und innerhalb des Körpergewebes mit vorzugsweise biodegradierbaren Leitungen versehen. Beispielsweise können von tief im Körperinneren liegenden Implantaten biodegradierbare Leitungen durch das Körpergewebe bis unter die Haut reichen. Um dann zum gewünschten Zeitpunkt die Polarisierung vorzunehmen, können wiederum wie beschrieben leitende Verbindungen in vergleichsweise einfacher Weise perkutan eingebracht werden, um mit der biodegradierbaren Leitung verbunden zu werden. Es ist somit möglich, das erfindungsgemäße Verfahren auch bei tiefliegenden Implantaten, welche perkutan nicht direkt erreichbar sind, einzusetzen. Da nach der Auflösung des Implantates auch die Leitungen degradieren, sind langfristig keine Abstoßungsprobleme oder anderen Reaktionen durch verbliebene Leitungen mehr zu erwarten. Die Beständigkeit der Leitungen sollte hierbei auf die Korrosionsrate des Implantates abgestimmt werden, um die Funktionalität des Implantatsystems zu gewährleisten. Ebenso kann bei schlecht zugänglichen Implantaten auch die Gegenelektrode implantiert werden und mit einer biodegradierbaren Leitung in die Nähe der Haut geführt werden.

Bei den letztgenannten Ausführungen bildet das Implantat die Arbeitselektrode, so dass das Implantat wahlweise anodisch oder kathodisch polarisiert werden kann, um die Korrosionsrate einerseits zu beschleunigen oder andererseits zu verringern. Ebenso kann bei der erstgenannten Ausführung der Erfindung, bei welcher keine direkte Kontaktierung und somit keine direkte leitende Verbindung einer Elektrode zu dem Implantat besteht, eine anodische Polarisierung des Implantates vorgenommen werden, in dem ein überwiegend anodischer Bereich in dem Implantat ausgebildet wird und somit die Korrosionsrate des Implantats beschleunigt wird. Es ist somit auch möglich, beispielsweise bei langsam korrodierenden Materialien nach Beendigung der Behandlung eine gezielte und schnelle Auflösung des Implantates vorzunehmen.

Ebenso kann auch ein überwiegend kathodischer Bereich in dem Implantat ausgebildet werden bzw. eine kathodische Polarisierung des Implantats vorgenommen werden, um die Korrosionsrate des Implantats zu verringern. Implantate, die in vivo unter freien Korrosionspotential zu schnell korrodieren, können hierdurch gezielt stabilisiert werden.

Vorteilhafterweise umfasst die Vorrichtung eine Steuereinheit, mittels welcher eine Stromstärke zwischen den Elektroden oder ein Potential zwischen den Elektroden einstellbar und/oder regelbar ist. Bei einem Verfahren zur Beeinflussung der Korrosionsrate von Implantatmaterialien in vivo wird der Stromfluss zwischen den Elektroden potentiostatisch oder galvanostatisch reguliert.

Bei dem erfindungsgemäßen Verfahren kann vorteilhafterweise die Korrosionsrate des Implantates durch die Höhe der angelegten Spannung oder des angelegten Stromes und/oder durch die Positionierung der Elektroden in Bezug auf das Implantat gesteuert werden. Ist das Implantat ausschließlich über Körpergewebe des Patienten mit den Elektroden verbunden, kann die Korrosionsrate sowohl durch die Positionierung des Implantates zwischen den beiden Elektroden als auch durch die Höhe der angelegten Spannung oder des angelegten Stromes gesteuert werden, so dass eine gezielte Steuerung der Korrosionsrate in besonders vorteilhafter Weise möglich ist. Eine besonders einfache Steuerung kann hierbei bereits durch ein einfaches Verschieben der Elektroden auf der Haut des Patienten erfolgen.

Besonders vorteilhaft ist es weiterhin, wenn die Höhe der angelegten Spannung oder des angelegten Stromes in Abhängigkeit von dem pH-Wert des Gewebes und/oder der Fließgeschwindigkeit des Elektrolyten und/oder der Position des Implantates in Bezug auf die Elektroden gewählt wird. Die Steuerung der Korrosionsrate ist hierdurch nicht vorbestimmt, sondern kann jederzeit auch nach Implantation entsprechend der vorliegenden Erfordernisse, beispielsweise bei Entzündungsvorgängen im Körper, oder in Abhängigkeit des Implantatortes gesteuert werden.

Besonders vorteilhaft ist es weiterhin, wenn die Höhe der angelegten Spannung oder des angelegten Stromes in Abhängigkeit eines Korrosionszustandes des Implantates in vivo oder in Abhängigkeit eines Krankheits/Genesungsbildes reguliert wird. So ist es beispielsweise möglich, den Zustand des Implantates oder eine nachwachsende oder sich neu bildende Gewebe- oder Gefäßstruktur mittels verschiedener bildgebender Verfahren zu kontrollieren und das Potential der Arbeitselektrode oder eine Stromstärke zwischen den Elektroden durch die Steuereinheit entsprechend zu regulieren.

Vorzugsweise ist wenigstens eine der Elektroden als inerte Elektrode aus einem biokompatiblen Werkstoff, beispielsweise Graphit, Titan oder Platin, ausgebildet. Durch die hohe chemische und elektrochemische Stabilität der inerten Elektrode ist sichergestellt, dass chemische Reaktionen an der Elektrode die Auflösung oder Stabilisierung des Implantates nicht beeinflussen.

Nach einer Weiterbildung der Erfindung umfasst die Vorrichtung weiterhin eine Referenzelektrode, welche über einen Elektrolyten und eine leitende Brücke mit dem Körpergewebe verbindbar ist oder direkt in das Körpergewebe implantierbar ist. Mittels eines Drei-Elektroden-Systems zur Durchführung des erfindungsgemäßen Verfahrens ist eine genaue Einstellung der Verfahrensparameter möglich. Die Brücke kann beispielsweise perkutan applizierbar sein. Die Referenzelektrode kann jedoch auch direkt implantiert werden, um eine bessere Verbindung mit dem Körpergewebe herzustellen.

Weitere Vorteile der Erfindung werden anhand der nachfolgend dargestellten Ausführungsbeispiele beschrieben. Es zeigen:
- **Figur 1**: eine erste Ausführung der Erfindung, bei welcher das Implantat ausschließlich durch Körpergewebe mit den beiden Elektroden verbunden ist,
- **Figur 2**: eine weitere Ausführung der Erfindung, bei welcher das Implan- tat ausschließlich durch Körpergewebe mit den Elektroden ver- bunden ist.
- **Figur 3**: eine alternative Ausführungsform der Erfindung mit einer galva- nostatischen Polarisierung des Implantates,
- **Figur 4**: eine Ausführung einer Vorrichtung mit einer Referenzelektrode, die sich außerhalb des Körpergewebes befindet,
- **Figur 5**: eine weitere Ausführung der Erfindung mit einer Referenzelekt- rode, die sich innerhalb des Körpergewebes befindet, sowie
- **Figur 6**: eine Ausführung der Erfindung für schwer zugängliche Implan- tate.

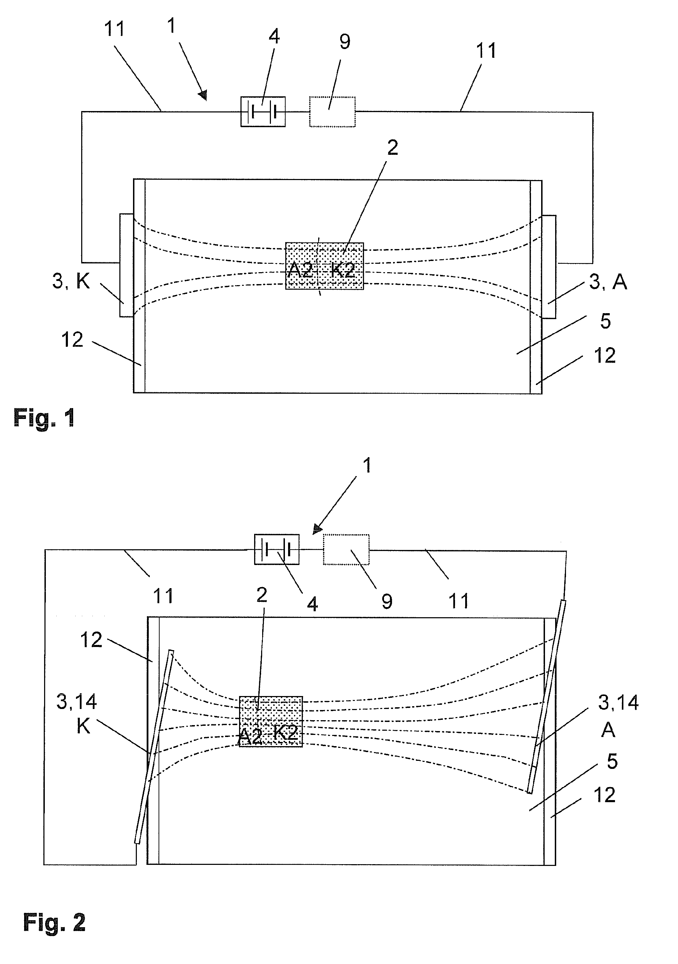

Fig. 1 zeigt eine erste Ausführung der Erfindung, bei welcher keine direkte Kontaktierung des Implantates 2 durch eine Elektrode 3 vorgesehen ist. Die Vorrichtung 1 umfasst zwei Elektroden 3, die über Leitungen 11 elektrisch leitend mit einer Strom- oder Spannungsquelle 4 verbunden sind. Innerhalb des Körpergewebes 5 eines Patienten liegt ein Implantat 2 aus einem metallischen, biokompatiblen und biokorrodierbaren Werkstoff. Die Elektroden 3 sowie die Strom- oder Spannungsquelle 4, welche als Gleichstrom- oder Gleichspannungsquelle ausgebildet ist, sind hierbei außerhalb des Körpers des Patienten angeordnet, so dass keinerlei invasive Eingriffe nach Implantation des Implantates 2 mehr erforderlich sind, um die Korrosionsrate des Implantates zu steuern. Nach der vorliegenden Darstellung sind die Elektroden 3 lediglich auf der Haut 12 des Patienten, beispielsweise als Klebeelektroden, angeordnet.

Die Elektroden 3 sind hierbei derart auf der Haut 12 des Patienten platziert, dass das Implantat 2 zwischen den Elektroden 3 zu liegen kommt und ein Stromkreis zwischen den Elektroden und dem Implantat ausschließlich über Körpergewebe 5 des Patienten geschlossen wird.

Erstaunlicherweise hat sich gezeigt, dass eine elektrochemische Zelle mit entsprechenden Reaktionen zur Auflösung oder Stabilisierung des Implantates auch dann gebildet werden kann, wenn das Implantat 2 nicht direkt über eine Leitung 11 mit einer Elektrode 3 verbunden wird. Es wurde herausgefunden, dass Streuströme (angedeutet durch strichpunktierte Linien zwischen den Elektroden), welche das Implantat 2 durchfließen, bereits ausreichend sind, um elektrochemische Reaktionen in dem Implantat 2 zu erzeugen. Durch den elektrischen Widerstand des Implantates 2 fällt an dem Implantat 2 Spannung ab und das Implantat 2 wird von Streuströmen des Stromflusses zwischen den Elektroden 3 durchflossen. Hierdurch wird in einem Teil des Implantates 2 ein anodischer Bereich A2 und in einem anderen Teil des Implantates 2, welcher der Anode A gegenüberliegt, ein kathodischer Bereich K2 ausgebildet.

Die Vorrichtung 1 bildet somit eine elektrochemische Zelle, durch welche bei gezieltem Anlegen eines Stromes oder einer Spannung die Korrosionsgeschwindigkeit des Implantates 2 in vivo gesteuert werden kann. Je nachdem, ob hierbei der anodische Bereich A2 oder der kathodische Bereich K2 überwiegt, kann das Implantat 2 gezielt elektrochemisch aufgelöst werden oder aber auch, beispielsweise bei Implantaten 2, welche unter freiem Korrosionspotential zu schnell korrodieren, eine gezielte Stabilisierung des Implantates 2 vorgenommen werden.

Da nach der in Figur 1 gezeigten Ausführung der Erfindung nach Implantation des Implantatsystems 1 die Hautbarriere des Patienten nicht durchbrochen eignet sich die dargestellte IVorrichtung 1 neben einer Kurzzeitanwendung auch zur Langzeitanwendung. Eine Infektionsgefahr wie bei invasiven Verfahren ist hierbei nicht gegeben. So ist es beispielsweise möglich, ein Implantat 2 aus einem Magnesium-Werkstoff, welches unter freiem Korrosionspotential sehr schnell korrodiert, über einen längeren Zeitraum zu stabilisieren. Die Lebensdauer eines Implantates 2 auf Magnesiumbasis im Körper des Patienten kann hierdurch erheblich verlängert werden. Die Vorrichtung 1 kann hierdurch auch tragbar ausgeführt werden, um vom Patienten im Alltag selbständig bedient zu werden.

Fig. 2 zeigt ebenso wie Fig. 1 eine Ausführung der Erfindung, wobei das Implantat 2 ausschließlich über Körpergewebe 5 des Patienten mit den Elektroden 3 verbunden ist. Die Elektroden 3 sind hierbei jedoch als Einstichnadeln 14 ausgebildet. In diesem Falle wird zwar die Hauptbarriere 12 des Patienten durchbrochen, es können jedoch ungünstig liegende Implantate in tieferen Gewebsschichten erreicht werden.

Bei dem erfindungsgemäßen Verfahren ist eine Steuerung der Korrosionsrate des Implantates 2 bereits dadurch möglich, dass die Elektroden 3 entsprechend in Bezug auf das Implantat 2 platziert werden. Versuche des Anmelders haben ergeben, dass die Aufteilung des Implantats in einen anodischen Bereich A2 und einen kathodischen Bereich K2 erheblich von dem Abstand des Implantats 2 von den Elektroden 3 abhängt. Untersuchungen des Anmelders, bei welchen Magnesiumproben in SBF (Simulated Body Fluids) entsprechend der Darstellungen der Fig. 1 und 2 platziert wurden, haben ergeben, dass eine maximale Abtragsrate in unmittelbarer Nähe der Anode A erreicht wird. Um die Abtragsrate gezielt einzustellen, sind daher verschiedenen Positionierungen der Elektroden 3 möglich, was die flexible Anwendbarkeit der Vorrichtung 1 weiter erhöht.

Werden also beispielsweise wie in Fig. 2 dargestellt, die Elektroden 3 derart platziert, dass das Implantat 2 nahe der negativ geladenen Elektrode bzw. Kathode K liegt, so bildet sich im Implantat 2 ein überwiegend kathodischer Bereich K2 aus, was zu einer Stabilisierung des Implantates führt. Eine derartige Anordnung ist daher beispielsweise zur Stabilisierung von Magnesiumimplantaten besonders geeignet. Ebenso kann allein durch die Positionierung der Elektroden 3 auch eine Auflösung von Implantaten 2 vorgenommen werden, wenn in dem Implantat 2 ein überwiegend anodischer Bereich ausgebildet wird.

Magnesiumwerkstoffe werden unter freiem Korrosionspotential in Körpergewebe mit einer Korrosionsrate von bis zu 60 mm pro Jahr abgetragen. Hierbei entsteht eine starke Korrosion, welche bereits nach kürzerer Zeit ein Implantat so schwächen kann, dass dieses seine Aufgabe nicht mehr erfüllen kann. Wird beispielsweise ein Magnesiumimplantat zur Fixierung von Knochenbrüchen eingesetzt, ist hierbei bedingt durch die starke lokale Korrosion eine Fixierung der Fraktur bereits nach vergleichsweise kurzer Zeit nicht mehr sicher gegeben.

Versuche des Anmelders zum Korrosionsverhalten von Magnesium, welche mit SBF (Simulated Body Fluids) als Elektrolyt zum Ersatz des Körpergewebes durchgeführt wurden, haben ergeben, dass durch gezielte kathodische Polarisierung eines Magnesiumimplantates die Abtragsraten erheblich reduziert werden können. So kann beispielsweise in entzündetem Körpergewebe (SBF mit pH 5,5) die jährliche Korrosionsrate um etwa den Faktor 4 gesenkt werden. Versuche des Anmelders haben gezeigt, dass der pH-Wert des Körpergewebes bzw. des SBF einen großen Einfluss auf die Korrosionsrate des Implantates hat. So wurden beispielsweise in normalem Körpergewebe bzw. SBF mit pH-Wert 7,4 unter freiem Korrosionspotential Korrosionsraten von etwa 10 mm pro Jahr ermittelt, kathodische Polarisierung des Implantates zeigt in diesem Fall keinen visuellen Korrosionsangriff. Weiterhin wurde ermittelt, dass die Zugabe von Albumin einen erheblichen Einfluss auf die Korrosionsrate hat.

Es ist somit durch gezielte kathodische Polarisierung bzw, durch gezielte Ausbildung eines überwiegend kathodischen Bereiches K2 in einem Implantat 2 auf Magnesiumbasis möglich, die Korrosionsrate in Abhängigkeit von Parametern wie pH-Wert und Zugabe von Albumin und entsprechend der gewünschten Behandlungsdauer bzw. Einsatzdauer des Implantates 2 einzustellen. Die in den Figuren 1 und 2 dargestellte Anordnung kann jedoch ebenso auch bei einem Implantat 2 auf Stahlbasis eingesetzt werden, um die Korrosionsrate gezielt zu beschleunigen und eine gleichmäßige Degradation des Implantates 2 zu erzielen.

Neben Versuchen zu den Auswirkungen der Position des Implantates 2 in Bezug auf die Elektroden 3 wurden weitere Untersuchungen nach den Anordnungen der Fig. 1 und 2 durchgeführt, um eine Abhängigkeit der korrosionsrate von der Höhe des angelegten Potentials oder der angelegten Ströme sowie der Fließgeschwindigkeit des Elektrolyten zu ermitteln. Die Versuche wurden hierbei mit Graphitelektroden durchgeführt, welche eine hohe chemische und elektrochemische Stabilität in SBF aufweisen.

Um den Einfluss der Höhe des angelegten Potentials bzw. des angelegten Stromes zu ermitteln, wurden die Untersuchungen in der Position des Implantates 2 in Bezug auf die Elektroden 3 durchgeführt, welche die geringste Abtragsrate aufwies. Die Versuche wurden mit Magnesium durchgeführt und das angelegte Potential wurde variiert im Bereich von -1,5 V bis -5,0 V bezogen auf eine Standard-Kalomel-Elektrode. Das freie Korrosionspotential von Magnesium liegt etwa bei -1,8 V bis -1,9 V bezogen auf die Standard-Kalomel-Elektrode. Es wurde hierbei herausgefunden, dass niedrige Potentialwerte von -3 V bis -5 V gegenüber der Standardelektrode eine wesentlich stärkere Korrosion und somit eine höhere Abtragsrate erzeugen. Es wurden jedoch bereits bei Potentialwerten von etwa -2V gegenüber der Standardelektrode gute Ergebnisse erzielt.

Bei einem erfindungsgemäßen Verfahren zur Steuerung der Korrosionsrate temporärer Implantate 2 in vivo ist es daher, um beispielsweise Magnesiumimplantate zu stabilisieren, ausreichend, Potentiale anzulegen, die nur wenig unter dem freien Korrosionspotential liegen, bzw. nur niedrige kathodische Ströme anzulegen.

Weiterhin wurden bei Versuchen mit Magnesium Flüssigkeitsbewegungen nachgestellt, um die Fließgeschwindigkeiten des Elektrolyten im Körper nachzustellen. Hierbei ergab sich, dass die Abtragsrate auch mit steigender Fließgeschwindigkeit zunimmt.

Um eine gezielte Beeinflussung der Korrosionsrate eines temporären Implantates 2 vorzunehmen, kann daher der Einfluss der Fließgeschwindigkeit durch eine entsprechend höhere oder niedrigere angelegte Spannung bzw. angelegte Ströme ausgeglichen werden. Die Höhe der angelegten Spannung bzw. des angelegten Stromes wird daher auch in Abhängigkeit des Einsatzortes des Implantates im Körper bzw. in Abhängigkeit der Fließgeschwindigkeit des Elektrolyten gewählt.

Weiterhin ergaben die Versuche, dass die Abtragsrate von Magnesium in bewegtem SBF bei Albuminzugabe auch bei niedrigen pH-Werten steigt. Das heißt, dass bei entzündetem Körpergewebe in diesem Falle eine schnellere Auflösung eines Magnesiumimplantates erfolgen kann, so dass wiederum die Höhe der angelegten Spannung bzw. des angelegten Stromes auch in Abhängigkeit des pH-Wertes bzw. der Anwesenheit von Albumin gewählt werden muss, um die Korrosionsrate in der gewünschten Höhe zu halten. Der beschriebene Effekt der Streustromkorrosion tritt hierbei unabhängig davon auf, ob die Medien bewegt oder unbewegt sind.

Um die Höhe des angelegten Potentials bzw. des angelegten Stromes zu regulieren, ist vorzugsweise eine Steuereinheit 9 vorgesehen, welche je nach Ausführung eine potentiostatische oder eine galvanostatische Regulierung vornehmen kann.

Obwohl bei der Ausführung der Erfindung nach den Figuren 1 und 2 lediglich ein Stromfluss durch Streuströme in dem Implantat 2 auftritt und diese allgemein als unerwünscht und nur schwer kontrollierbar betrachtet werden, kann erstaunlicherweise ein sehr genaue Steuerung der Korrosionsrate des Implantats 2 vorgenommen werden.

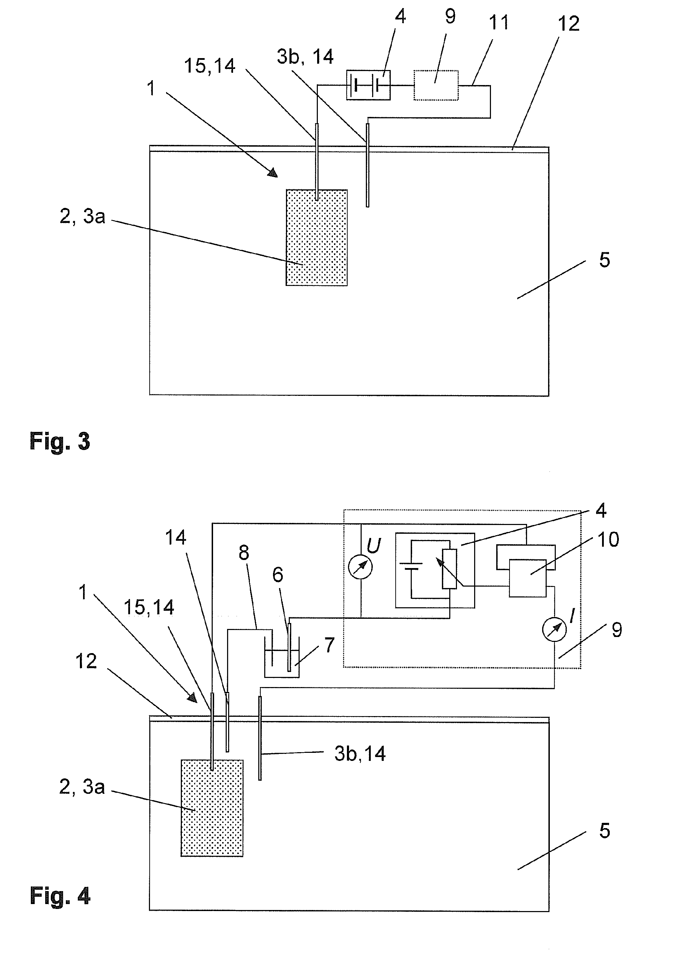

Figur 3 zeigt eine andere Ausführungsform der Erfindung, bei welcher das Implantat als Arbeitselektrode 3a fest implantiert ist. Die Stromquelle 4 ist außerhalb des Körpers angeordnet und eine elektrisch leitende Verbindung 15 ist in direkt leitendem Kontakt zum Implantat 2 angeordnet. Das Implantat 2 ist hierbei über Körpergewebe 5 als Elektrolyt mit einer Gegenelektrode 3b verbunden. Die Gegenelektrode 3b ist aus einem inerten Material wie Grafit, Platin oder Titan hergestellt. Die elektrisch leitende Verbindung 15 sowie die Gegenelektrode 3b sind als Einstichnadeln 14 ausgebildet und perkutan in das Körpergewebe 5 des Patienten appliziert. Hierbei ist es auch möglich, tief unter der Haut angeordnete Implantate 2 zu erreichen.

Besonders vorteilhaft ist es auch hier, dass lediglich das Implantat 2 implantiert werden muss und zu jedem beliebigen gewünschten Zeitpunkt nach Implantation durch das perkutane Anbringen der elektrisch leitenden Verbindung 15 und der Gegenelektrode 3b eine gezielte Steuerung der Korrosionsrate des Implantates 2 von außen erfolgen kann. Die Vorrichtung 1 weist eine Steuereinheit 9 mit einem Galvanostaten auf, mittels welcher die jeweils gewünschte Stromstärke einstellbar ist. Somit ist es möglich, auf Abweichungen von den gegebenen Bedingungen zu reagieren und die angelegte Stromstärke entsprechend zu erhöhen oder zu erniedrigen, um die ungeplant auftretenden Einflüsse auszugleichen.

Da hierbei die schützende Hautbarriere durchbrochen wird, besteht die Gefahr, dass Keime von den außenliegenden Teilen der Elektroden 3a, 3b sich in das Körperinnere ausbreiten, so dass eine erhöhte Infektionsgefahr besteht. Diese Anwendung ist somit vor allem zur Anwendung über einen vergleichsweise kurzen Zeitraum geeignet. Beispielsweise ist es möglich, Implantate 2 aus Eisenwerkstoffen durch eine anodische Polarisierung aufzulösen.

Versuche des Anmelders in SBF haben hierbei ergeben, dass die Korrosionsrate von Eisen durch gezielt angelegte Spannung oder Strom im anodischen Bereich erheblich erhöht werden kann. Auch hier wurden erhebliche Einflüsse des pH-Wertes sowie der Zugabe von Albumin auf die Korrosionsrate festgestellt. Insbesondere in entzündetem Körpergewebe (SBF mit pH-Wert 5,5) konnte ein erheblicher Einfluss der Albuminkonzentration festgestellt werden, welcher den Korrosionsangriff beschleunigte. Weiterhin wurde für entzündetes Körpergewebe ein eher gleichmäßiges Korrosionsbild ermittelt, während bei höherem pH-Werten eine eher lokale Korrosion auftrat.

Da die Korrosionsrate bei Eisenwerkstoffen erheblich erhöht werden kann, ist es somit mit der in Figur 3 beschriebenen Anordnung möglich, nach Ende der Behandlung das Implantat 2 durch galvanostatische Anodisierung in vergleichsweise kurzer Zeit und bei gleichmäßigem Abtrag aufzulösen.

Figur 4 zeigt eine weitere Ausführung der erfindungsgemäßen Vorrichtung 1 in einer Drei-Elektroden-Anordnung. Neben der Arbeitselektrode 3a, der Gegenelektrode 3b und der Spannungs- oder Stromquelle 4 umfasst die Vorrichtung 1 weiterhin eine Referenzelektrode 6, welche ebenfalls über das Körpergewebe 5 leitend mit der Arbeitselektrode 3a in Verbindung steht. Die Verbindung der Referenzelektrode 6 mit dem Körpergewebe 2 erfolgt hierbei über einen Elektrolyten 7 und eine Brücke 8. Wie aus der Darstellung ersichtlich, sind hierbei die Brücke 8, die Gegenelektrode 3b sowie die leitende Verbindung 15 zum Implantat 2 als Einstichnadeln 14 ausgebildet. Als Referenzelektrode 6 ist beispielsweise eine gesättigte Kalomel-Elektrode einsetzbar.

Mittels der gezeigten Drei-Elektroden-Anordnung ist neben der bereits beschriebenen galvanostatischen Polarisierung auch eine potentiostatische Polarisierung des Implantats 2 möglich. Ebenfalls möglich ist eine potentiostatische Polarisierung außerhalb des Körpers, wie in Figur 3 dargestellt, zur Auflösung von Metallschäumen, z. B. aus Eisen, unlegierten Stählen und Wolfram.

Um die Polarisierung des Implantats 2 bzw. die Ausbildung entsprechender Bereiche gezielt durchführen zu können, ist es sowohl bei galvanostatischer Polarisierung wie auch bei potentiostatischer Polarisierung vorteilhaft, wenn die Vorrichtung 1 eine Steuereinheit 9 umfasst. Mittels der Steuereinheit 9 ist dann die Stromstärke bzw. das Potential zwischen den Elektroden 3 regulierbar.

Nach der Darstellung der Figur 4 beinhaltet die Steuereinheit 9 einen Potentiostaten 10, mittels welchem die Polarisierung des Implantats 2 regulierbar ist. Vorzugsweise ist an der Spannungsquelle 4, wie hier dargestellt, eine Sollspannung einstellbar. Die Sollspannung kann beispielsweise je nach verwendetem Implantatmaterial, dem Zustand des Implantates 2 in vivo, dem Behandlungsfortschritt oder in Abhängigkeit weiterer Parameter eingestellt werden. Es ist jedoch auch möglich, eine Spannungs quelle 4 mit fester Spannung einzusetzen.

Potentiostatische Untersuchungen des Anmelders zur Abtragung von Stahl haben beispielsweise bei einem Potential von 0 V gegenüber der Referenz-Kalomel-Elektrode 6 Abtragsraten von ca. 70 mm pro Jahr ergeben. Eine Beeinflussung der Abtragsrate ist hierbei durch die Einstellung des Potentials an der Arbeitselektrode 3a möglich, so dass die Lebensdauer des Implantates 2 in vivo vergleichsweise genau auf die Bedürfnisse abgestimmt werden kann. Für die Stabilisierung eines Magnesiumimplantates hat sich ein Potential von etwa -2 V gegenüber der Referenzelektrode 6 als vorteilhaft erwiesen. Es versteht sich, dass der beschriebene Aufbau mit einem Potentiostaten in analoger Weise auch in den Vorrichtungen nach den Figuren 1 oder 2 verwendet werden kann.

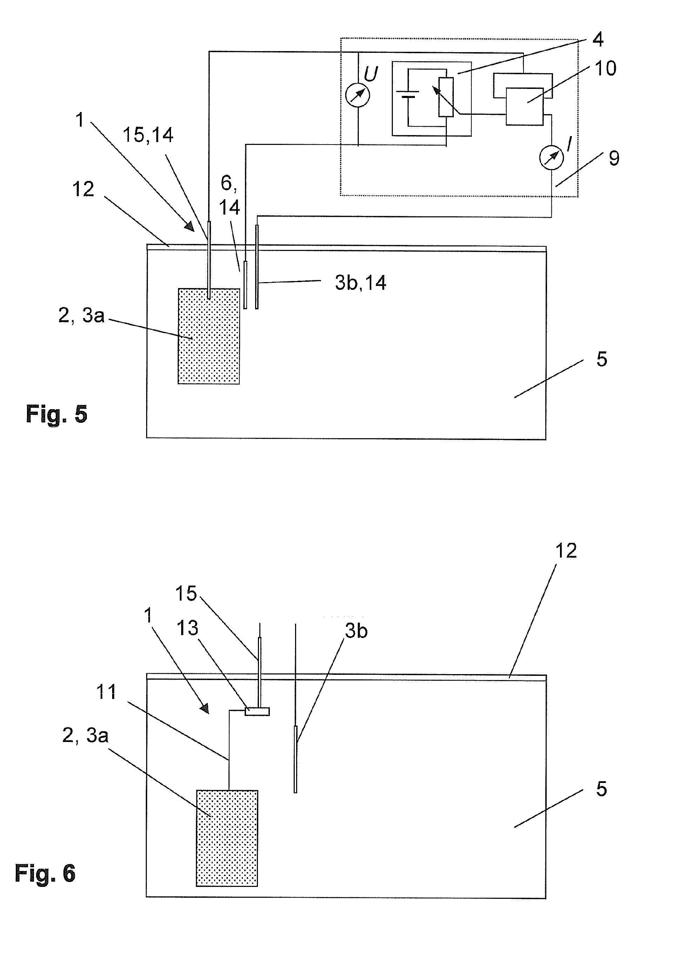

Figur 5 zeigt im Wesentlichen die Ausführung der Erfindung nach Figur 4, wobei jedoch die Referenzelektrode 6 sich ebenfalls innerhalb des Körpergewebes befindet. Eine Brücke sowie ein externer Elektrolyt sind hierbei nicht erforderlich, was die Handhabung des Implantatsystems erleichtert. Die Referenzelektrode 6 ist hierzu ebenfalls perkutan applizierbar oder kann ggf. sogar implantiert sein.

Figur 6 zeigt eine weitere Ausführungsform der Erfindung, welche insbesondere bei tief liegenden, schlecht erreichbaren Implantaten 2 einsetzbar ist. So kann beispielweise ein tief liegendes Implantat 2, mit einer Leitung 11 versehen werden, welche ebenfalls implantierbar ist. Diese kann unterhalb der Haut 12 einen Anschluss 13 beinhalten. Die leitende Verbindung 15 von einer externen Spannungs- oder Stromquelle 4 (hier nicht dargestellt) zu dem Implantat 2 kann ebenso wie die Gegenelektrode 3b als Einstichnadel 14 im Bereich der Leitung 11 bzw. des Anschlusses 13 eingebracht werden.

Zum Entfernen der Leitung 11 und des Anschlusses 13 ist dann nur ein kleiner Schnitt erforderlich , welcher keine große Belastung des Patienten darstellt. Besonders vorteilhaft ist es jedoch, wenn wenigstens die Leitung 11 und ggf. auch der Anschluss 13 aus einem biodegradierbaren Material hergestellt sind. Diese müssen somit wie das Implantat 2 nicht entfernt werden, so dass auch hierdurch die Belastungen des Patienten weiter reduziert werden können. Die Korrosionsrate der biodegradierbaren Leitungen 11 sowie des Anschlusses 13 ist hierbei auf die Korrosionsrate des Implantates 2 abzustimmen, so dass eine leitende Verbindung für die Beeinflussung der Korrosionsrate gewährleistet ist. Dies ist ggf. durch Abstimmung der Materialien sowie bestimmter Beschichtungen möglich.

Die Erfindung ist nicht auf die dargestellten Ausführungsbeispiele beschränkt. So ist beispielsweise anstelle von Eisenwerkstoffen und Magnesium eine Vielzahl von weiteren Implantatwerkstoffen möglich, welche gezielt in ihrem Korrosionsverhalten beeinflusst werden können. Weiterhin sind als Implantate nicht nur Knochenimplantate, sondern verschiedenste temporäre Implantate möglich. So ist es beispielsweise auch möglich, Stents aus einem Magnesiummaterial zu fertigen und durch die kathodische Polarisierung bzw. die Ausbildung eines überwiegend kathodischen Bereiches zu stabilisieren. Die Korrosionsgeschwindigkeit kann hierbei vorteilhafterweise derart eingestellt werden, dass je nach Einsatzzweck des Stents und weiteren Gegebenheiten eine vollständige Degradation bereits nach wenigen Wochen erfolgt. Ist es erforderlich, dass Gefäßlumen über einen längeren Zeitraum abzustützen, so kann das Implantat auch über einen längeren Zeitraum entsprechend den medizinischen Erfordernissen stabilisiert werden.

Auch ist es möglich, die Form des Implantats entsprechend der gewünschten Verweildauer im Körper anzupassen. Beispielsweise kann das Implantat an die medizinisch notwendige Struktur angrenzend einen Bereich aufweisen, der nur für die elektrochemische Reaktion vorgesehen ist. Durch den Stromfluss durch das Implantat wird dann in der medizinisch notwendigen Struktur ein kathodischer Bereich ausgebildet, während der zusätzliche Bereich eine eine Art "Opferanode" bildet. Die medizinisch notwendige Struktur kann hierdurch geschützt werden.

Weiterhin ist die Erfindung für die verschiedensten temporären chirurgischen oder orthopädischen Implantate einsetzbar. Weitere Abwandlungen und Kombinationen im Rahmen der Patentansprüche fallen ebenfalls unter die Erfindung.

### Bezugszeichenliste

- 1: Vorrichtung
- 2: Implantat
- 3: Elektroden
- 3a: Arbeitselektrode
- 3b: Gegenelektrode
- 4: Spannungs- oder Stromquelle
- 5: Körpergewebe
- 6: Referenzelektrode
- 7: Elektrolyt
- 8: Brücke
- 9: Steuereinheit
- 10: Potentiostat
- 11: Leitung
- 12: Haut
- 13: Anschluss
- 14: Einstichnadeln
- 15: elektrisch leitende Verbindung
- A: Anode
- K: Kathode
- A2: anodischer Bereich
- K2: kathodischer Bereich

## Patentansprüche

1. Verfahren zur Beeinflussung der Korrosionsrate von Implantaten (2) aus einem metallischen, biokompatiblen und biokorrodierbaren Werkstoff in vivo, bei welchem eine elektrochemische Zelle mit Körpergewebe (5) des Patienten als Elektrolyt aufgebaut wird, **dadurch gekennzeichnet, dass**
- nach Implantation eines Implantats (2) eine gezielte Steuerung einer Auflösung oder Stabilisierung des Implantats (2) erfolgt,
- wobei eine Spannungs- oder Stromquelle (4) außerhalb des Körpers des Patienten vorgesehen wird und
- wenigstens eine Elektrode (3) und/oder eine elektrisch leitende Verbindung (15) an dem oder in das Körpergewebe (5) appliziert wird und
- die wenigstens eine Elektrode (3) und/oder die elektrisch leitende Verbindung (15) entweder direkt kontaktierend oder ausschließlich über Körpergewebe (5) des Patienten mit dem Implantat (2) verbunden wird.

2. Verfahren nach dem vorhergehenden Anspruch, **dadurch gekennzeichnet, dass**
- außerhalb des Körpers des Patienten zwei Elektroden (3) vorgesehen werden, die an eine Spannungs- oder Stromquelle (4) angeschlossen sind,
- die Elektroden (3) nach Implantation des Implantats (2) vorzugsweise auf der Haut (12) des Patienten derart platziert werden, dass das Implantat (2) zwischen den beiden Elektroden (3) liegt, - dass die beiden Elektroden (3) ausschließlich über Körpergewebe (5) des Patienten mit dem Implantat (2) verbunden werden und
- dass die Auflösung oder Stabilisierung des Implantats (2) ohne direkte Kontaktierung ausschließlich durch Streuströme erfolgt, welche das Implantat (2) durchfließen.

3. Verfahren nach Anspruch 1, **dadurch gekennzeichnet, dass**
- die Spannungs- oder Stromquelle (4) über eine elektrisch leitende Verbindung (15) direkt kontaktierend mit dem Implantat (2) als Arbeitselektrode (3a) und einer zweiten Elektrode (3) als Gegenelektrode (3b) verbunden wird,
- dass die Arbeitselektrode (3a) und die Gegenelektrode (3b) über Körpergewebe (5) des Patienten als Elektrolyt verbunden werden, und
- dass die Gegenelektrode (3b) und die elektrisch leitende Verbindung (15) nach Implantation des Implantats (2) perkutan in das Körpergewebe (5) eingebracht werden.

4. Verfahren nach einem der vorhergehenden Ansprüche, **dadurch gekennzeichnet, dass** der Stromkreis der elektrochemischen Zelle nach Implantation geschlossen wird.

5. Verfahren nach Anspruch 2, **dadurch gekennzeichnet, dass** durch die angelegte Spannung oder den angelegten Strom zwischen den Elektroden (3) ein Stromfluss durch das Implantat (2) hervorgerufen wird und dass durch den Stromfluss ein anodischer und ein kathodischer Bereich (A2, K2) in dem Implantat ausgebildet wird.

6. Verfahren nach einem der vorhergehenden Ansprüche, **dadurch gekennzeichnet, dass** das Implantat (2) gezielt anodisch polarisiert wird oder gezielt ein überwiegend anodischer Bereich (A2) in dem Implantat (2) ausgebildet wird und die Korrosionsrate des Implantats (2) hierdurch erhöht wird.

7. Verfahren nach einem der vorhergehenden Ansprüche, **dadurch gekennzeichnet, dass** das Implantat (2) gezielt kathodisch polarisiert wird oder gezielt ein überwiegend kathodischer Bereich (K2) in dem Implantat (2) ausgebildet wird und die Korrosionsrate des Implantats (2) hierdurch verringert wird.

8. Verfahren nach einem der vorhergehenden Ansprüche, **dadurch gekennzeichnet, dass** der Stromfluss zwischen den Elektroden (3) galvanostatisch oder potentiostatisch reguliert wird.

9. Verfahren nach einem der vorhergehenden Ansprüche, **dadurch gekennzeichnet, dass** die Korrosionsrate des Implantats (2) durch die Positionierung der Elektroden (3) in Bezug auf das Implantat (2) und/oder die Höhe der angelegten Spannung oder des angelegten Stromes gesteuert wird.

10. Verfahren nach einem der vorhergehenden Ansprüche, **dadurch gekennzeichnet, dass** die Höhe der angelegten Spannung oder des angelegten Stromes in Abhängigkeit von dem pH-Wert des Gewebes (5) und/oder der Fliessgeschwindigkeit des Elektrolyten und/oder der Position des Implantates (2) in Bezug auf die Elektroden (3) gewählt wird.

11. Verfahren nach einem der vorhergehenden Ansprüche, **dadurch gekennzeichnet, dass** die Höhe der angelegten Spannung oder des angelegten Stromes in Abhängigkeit eines Korrosionszustandes des Implantats (2) in vivo und/oder eines Krankheits/Genesungsbildes reguliert wird.

12. Verfahren nach einem der vorhergehenden Ansprüche, **dadurch gekennzeichnet, dass** dem Implantat (2) eine Referenzelektrode (6) zugeordnet wird, welche nach Implantation der Arbeitselektrode (3a) über eine Brücke (8) perkutan mit dem Körpergewebe (5) verbunden wird oder direkt implantiert wird.

13. Vorrichtung (1) zur Beeinflussung der Korrosionsrate eines Implantats (2) aus einem metallischen, biokompatiblen und biokorrodierbaren Werkstoff in vivo mit zwei Elektroden (3), welche jeweils elektrisch leitend mit einer Strom- oder Spannungsquelle (4) verbunden sind, **dadurch gekennzeichnet,**
- **dass** eine Auflösung oder Stabilisierung des Implantats (2) nach Implantation des Implantats (2) von außerhalb des Körpers des Patienten gezielt steuerbar ist,
- wobei die Strom- oder Spannungsquelle (4) außerhalb des Körpers des Patienten angeordnet ist und
- wenigstens eine Elektrode (3) und/oder eine elektrisch leitende Verbindung (15) von außen an dem oder in das Körpergewebe (5) applizierbar ist,
- und die wenigstens eine Elektrode (3) und/oder die elektrisch leitende Verbindung (15) entweder direkt kontaktierend oder ausschließlich über Körpergewebe (5) des Patienten mit dem Implantat (2) verbunden ist.

14. Vorrichtung nach dem vorhergehenden Anspruch, **dadurch gekennzeichnet, dass**
- die zwei Elektroden (3) vorzugsweise außerhalb des Körpers des Patienten derart angeordnet sind, dass das Implantat (2) in vivo zwischen den beiden Elektroden (3) liegt, und
- dass das Implantat (2) ohne direkte Kontaktierung ausschließlich über Körpergewebe (5) des Patienten mit den beiden Elektroden (3) verbunden ist, um einen Stromkreis der elektrochemischen Zelle nach Implantation zu schließen.

15. Vorrichtung nach Anspruch 13, **dadurch gekennzeichnet, dass**
- das Implantat (2) nach Implantation die erste Elektrode (3) als Arbeitselektrode (3a) bildet und über eine elektrisch leitende Verbindung (15) direkt mit der Strom- oder Spannungsquelle (4) verbunden ist,
- dass das Implantat (2) über Körpergewebe (5) des Patienten als Elektrolyt mit der zweiten Elektrode (3) als Gegenelektrode (3b) verbunden ist und
- dass die Gegenelektrode (3b) und die elektrisch leitende Verbindung (15) perkutan in das Körpergewebe (5) applizierbar sind, um einen Stromkreis der elektrochemischen Zelle nach Implantation zu schließen.

16. Vorrichtung nach einem der vorhergehenden Ansprüche, **dadurch gekennzeichnet, dass** das Implantat (2) ein temporäres Implantat (2) ist.

17. Vorrichtung nach einem der vorhergehenden Ansprüche, **dadurch gekennzeichnet, dass** das Implantat (2) eine Anode (A) der elektrochemischen Zelle bildet oder einen überwiegend anodischen Bereich (A2) aufweist, um die Korrosionsrate des Implantats (2) zu erhöhen.

18. Vorrichtung nach einem der vorhergehenden Ansprüche, **dadurch gekennzeichnet, dass** das Implantat (2) eine Kathode (K) der elektrochemischen Zelle bildet oder einen überwiegend kathodischen Bereich (K2) aufweist, um die Korrosionsrate des Implantats (2) zu verringern.

19. Vorrichtung nach einem der vorhergehenden Ansprüche, **dadurch gekennzeichnet, dass** die Vorrichtung (1) eine Steuereinheit (9) umfasst, mittels welcher eine Stromstärke zwischen den Elektroden (3) oder ein Potential zwischen den Elektroden (3) einstellbar und/oder regelbar ist.

20. Vorrichtung nach einem der vorhergehenden Ansprüche, **dadurch gekennzeichnet, dass** die Korrosionsrate durch die Positionierung des Implantats (2) in Bezug auf die Elektroden (3) und/oder durch die Höhe der angelegten Spannung oder des angelegten Stromes steuerbar ist.

21. Vorrichtung nach einem der vorhergehenden Ansprüche, **dadurch gekennzeichnet, dass** wenigstens eine der Elektroden (3) eine inerte Elektrode (3) aus einem biokompatiblen Werkstoff ist.

22. Vorrichtung nach einem der vorhergehenden Ansprüche, **dadurch gekennzeichnet, dass** die Vorrichtung (1) eine Referenzelektrode (6) umfasst, welche über eine Brücke (8) mit dem Körpergewebe (5) verbindbar ist oder direkt implantierbar ist.

23. Vorrichtung nach einem der vorhergehenden Ansprüche, **dadurch gekennzeichnet, dass** das Implantat (2) und/oder die Gegenelektrode (3b) implantierbar und innerhalb des Körpergewebes (5) mit vorzugsweise biodegradierbaren Leitungen (11) versehen sind.
